# EUROPEAN PATENT SPECIFICATION

(11) **EP 3 791 186 B1**
(45) Date of publication and mention of the grant of the patent: **24.01.2024**
(21) Application number: 19724265.4
(22) Date of filing: 03.05.2019
(51) Int. Cl.: G01N 33/574

(54) **ANTIGEN BIOMARKER OF MICROSATELLITE INSTABILITY**
ANTIGEN-BIOMARKER DER MIKROSATELLITENINSTABILITÄT
BIOMARQUEUR ANTIGÉNIQUE DE L'INSTABILITÉ MICROSATELLITAIRE

(30) Priority: 03.05.2018 PT 2018110722
(43) Date of publication of application: 17.03.2021
(73) Proprietor: Instituto de Patologia e Imunologia da Universidade do Porto (IPATIMUP), 4200-135 Porto (PT)
(72) Inventor: ALBUQUERQUE REIS, Celso, 4200-135 Porto (PT); MEREITER, Stefan, 4200-135 Porto (PT); RODRIGUES LEITE DE MAGALHÃES, Ana Maria, 4200-135 Porto (PT); POLOM, Karol, 53100 Siena (IT); ROVIELLO, Franco, 53100 Siena (IT)
(74) Representative: Ferreira Pinto, Francisca
(86) International application number: PCT/PT2019/050009
(87) International publication number: WO 2019/212373

(56) References cited:
- CLAUSEN H ET AL: "Monoclonal antibodies directed to the blood group a associated structure, galactosyl-A: Specificity and relation to the thomsen-friedenreich antigen", MOLECULAR IMMUNOLOGY, PERGAMON, GB, vol. 25, no. 2, 1 February 1988 (1988-02-01), pages 199-204, XP023681559, ISSN: 0161-5890, DOI: 10.1016/0161-5890(88)90068-5 [retrieved on 1988-02-01] cited in the application
- HIRONORI KUMAGAI ET AL: "Multifunctional nanobeacon for imaging Thomsen-Friedenreich antigen-associated colorectal cancer", INTERNATIONAL JOURNAL OF CANCER, vol. 132, no. 9, 1 May 2013 (2013-05-01), pages 2107-2117, XP055601126, US ISSN: 0020-7136, DOI: 10.1002/ijc.27903
- DIETMAIER WOLFGANG ET AL: "Diagnostic microsatellite instability: Definition and correlation with mismatch repair protein expression", CANCER RESEARCH, AMERICAN ASSOCIATION FOR CANCER RESEARCH, US, vol. 57, no. 21, 1 November 1997 (1997-11-01), pages 4749-4756, XP002199560, ISSN: 0008-5472
- F. SANTOS-SILVA ET AL: "Thomsen-Friedenreich antigen expression in gastric carcinomas is associated with MUC1 mucin VNTR polymorphism", GLYCOBIOLOGY, vol. 15, no. 5, 15 December 2004 (2004-12-15), pages 511-517, XP055600873, US ISSN: 0959-6658, DOI: 10.1093/glycob/cwi027
- STEFAN MEREITER ET AL: "The Thomsen-Friedenreich Antigen: A Highly Sensitive and Specific Predictor of Microsatellite Instability in Gastric Cancer", JOURNAL OF CLINICAL MEDICINE, vol. 7, no. 9, 5 September 2018 (2018-09-05), page 256, XP055601081, DOI: 10.3390/jcm7090256

## Description

### Technical field of the invention

The present invention refers to the use of antibodies or other agents with affinity for the Thomsen-Friedenreich (TF) antigen in a composition for *in vitro* diagnostics of Microsatellite Instability (MSI) through a method that comprises incubating the said composition employing the anti-TF antigen antibodies and assessing the expression of the TF antigen in a biological sample. A kit comprising the above mentioned compositions for detecting MSI by conducting the present invention's method is also disclosed.

The use, composition, methods and kit of the present invention may be advantageously applied in carcinoma of gastric, colorectal and other tissues to significantly facilitate the identification of MSI in the clinical setting through a single marker-based rapid, sensitive and specific antibody-assay for diagnostics, prognostics or prediction of response to treatment in cancer patients.

Thus, the present invention falls within the technical field of medicine, pharmaceutics and biochemistry.

### State of the art

Gastric cancer is a heterogeneous disease that requires multidisciplinary treatment [1]. Currently, new molecular classifications for gastric cancer have been proposed by TCGA (The Cancer Genome Atlas) and ACRG (Asian Cancer Research Group) in which microsatellite instability (MSI) is described as distinct subgroup [1, 2].

MSI is thus a distinct molecular subtype of gastric cancer and according to TCGA, the MSI subgroup is linked with hypermutation, gastric CpG island methylator phenotype, MLH1 silencing, and mitotic pathways [1]. DIETMAIER WOLFGANG ET AL: "Diagnostic microsatellite instability: Definition and correlation with mismatch repair protein expression",CANCER RESEARCH, AMERICAN ASSOCIATION FOR CANCER RESEARCH, US, vol. 57, no. 21, 1 November 1997 (1997-11-01), pages 4749-4756, recommends the use of a panel of 10 microsatellites and a definition of at least 40% instability (using these defined marker loci) in the diagnostic analysis of MSI.

In recent years, the clinical consequences of MSI and the therapeutic opportunities to target this peculiar cancer subtype became evident. Many publications described MSI from a clinical point of view, being found in 5.6% to 33.3% of all gastric cancers, and strongly associated with female sex, older age, intestinal histotype, middle/lower gastric position, N0 status, and TNM stage I/II with favorable overall survival [1-5].

It is important to underline that MSI is not a homogenous group and it has been shown that MSI is a prognostic factor because of the association with tumor localization and Lauren classification [6].

Additionally, it has been found that MSI is linked with PD-L1 expression and several specific drugs are currently used for clinical management of gastric cancer targeting the PD1/PD-L1 immune checkpoint pathway [7].

The diagnosis of MSI gastric cancer is of highest importance, especially before multidisciplinary treatment. It presents different responses to neoadjuvant chemotherapy and requires specific surgical treatment in synchronous metastases, includes the possibility of tailored lymphadenectomy, and stratifies for the application of targeted therapies [3, 8].

The transformation of epithelial cells is accompanied by several changes in the protein glycosylation machinery resulting in the aberration of cellular glycosylation, such as the truncation of O-linked glycans and the expression of sialofucosylated glycan epitopes [9]. The truncation of O-linked glycans has been widely described in various gastrointestinal carcinomas and results in the de novo expression of short O-glycans such as the Thomsen-Friedenreich (TF or T, Galβ1-3GalNAcα1) antigen, Thomsen-nouvelle (Tn, GalNAcα1) and its sialylated form sialyl Tn (STn, Neu5Acα2-3GalNAcα1) [9]. In addition, the expression of complex sialofucosylated structures, such as sialyl-Lewis A (SLea, Neu5Acα2-3Galβ1-3[Fucα1-4]GlcNAcβ-) and sialyl-Lewis X (SLex, Neu5Acα2-3Galβ1-4[Fucα1-3]GlcNAcβ-) has been frequently described in gastric tumors with important role in cancer progression and metastasis formation [9].

The TF antigen, also known as core 1 structure, is an intermediate structure during the maturation of mucin-type O-glycans within the Golgi apparatus. Under physiologic conditions, the TF antigen is below the limit of detection because it is modified with additional saccharides and made inaccessible due to surrounding larger glycans [10, 11]. It is only found in the luminal surfaces of the pancreatic duct, kidney distal tubule and kidney collecting duct [11] . In addition, macrophages of the thymus, spleen and lymph nodes carry the TF antigen, suggesting a potential immunologic role [11]. Importantly, the TF antigen is neither found in the healthy gastric epithelium nor in gastric pre-malignant conditions [12]. In gastric cancer the TF antigen is expressed in considerable amounts in around 210 of the tumours, associating with higher immune response [12].

As molecules that are secreted into circulation and due to their expression specificity for malignant cells, aberrant glycans and glycoconjugates have a long lasting history as cancer biomarkers [13] .

The TF antigen has several additional assets which could allow it to be used as serologic biomarker application as it is hardly expressed in human tissues, neither under healthy nor under other pathologic conditions [11,14]. Secondly, the basal levels of exposed TF are very low in the blood stream due to naturally circulating anti-TF IgM and IgG antibodies and the rapid clearance of terminally galactosylated glycoconjugates by the liver [15] . Lastly, as a mucin-type O-glycosylation it may be carried by a wide range of secreted glycoproteins that are overexpressed in gastric cancer such as MUC1 or CD44.

Usually, MSI/dMMR status is also tested in colorectal cancer specimens for the identification of patients at elevated risk for Lynch syndrome as well as for prognostic stratification. However, recent data has emerged showing that MSI/dMMR can have predictive value for immune checkpoint inhibitor therapy, regardless of the cancers' tissue of origin [16] . In fact, it was approved by the Food and Drug Administration that pembrolizumab (anti-programmed cell death protein-1 (PD-1)) could be used in any solid tumor with MSI/dMMR that have progressed following prior treatment.

Currently, one method of performing the MSI assay is performed by tumor and constitutional DNA extraction. Mononucleotide repeats are evaluated with fluorescently labelled Polymerase Chain Reaction (PCR) primers for BAT-26, NR-21, BAT-25, NR-27 and NR-24 in an equipment such as the ABI PRISM from Applied Biosystems. Repeats are co-amplified on tumor and matched constitutional DNA of each patient in a pentaplex PCR using the protocol for multiplex PCR disclosed by Qiagen. The allelic profiles are detected by the automated DNA sequencer ABI PRISM 3100 Genetic Analyzer from Applied Biosystems and the MSI status is determined in accordance to the guidelines of the National Cancer Institute on MSI for cancer detection and familial predisposition: whenever 2 or more markers show instability out of the 5 loci, the tumor is considered MSI high. In contrast, when one or no locus is instable, MSI low or Micro Satellite Stability is diagnosed, respectively.

Mismatch repair deficiency (dMMR) testing can also be performed by immunohistochemistry in paraffin sections using commercially available antibodies evaluating the expression of four MMR proteins (MLH1, PMS2, MSH2 and MSH6) in tumor cell nuclei.

Thus, current MSI/dMMR analysis are resource intensive procedures evaluating either the tumor PCR profile of 5 MSI markers and comparing them to the profile of matching normal DNA or evaluating the absence of at least one of 4 nuclear expressed markers in tumor sections.

Overall, despite the importance of MSI for the stratification of patients, the time and resources required for diagnosis of MSI still presents an obstacle and assay based on a single biomarker is known in the art to detect this feature.

### Summary of the Invention

The present invention refers to the use of an antibody with affinity for the Thomsen-Friedenreich (TF) antigen as a reagent for in vitro diagnostics of Microsatellite Instability (MSI).

Disclosed but not claimed is also a reagent composition for *in vitro* diagnostics of MSI comprising an antibody with affinity for the TF antigen.

In one embodiement, the composition comprises the anti-TF antigen antibody 3C9.

In another embodiement, the composition comprises the anti-TF antigen antibody SPM320.

In another embodiement, the composition comprises the anti-TF antigen antibody A68/B-A11.

In another embodiement, the composition comprises the anti-TF antigen antibody A78/G-A7.

In another embodiement, the composition comprises the anti-TF antigen antibody B79-H/B8.

In another embodiement, the composition comprises the anti-TF antigen antibody C80-I/C9.

In another embodiement, the composition comprises the anti-TF antigen antibody A68-B/A11.

In another embodiement, the composition comprises a fragment of the variable domain of the previously mentioned anti-TF antibodies.

In another embodiement, the composition comprises a lectin with affinity for the TF antigen, for example Jacalin.

In another embodiement, the composition further comprises at least one of the following components: a buffer, a blocking agent, a preservative or mixtures thereof.

Disclosed but not claimed is also a kit for *in vitro* diagnostics of MSI comprising at least one of the above described compositions.

The said kit further comprises control and/or calibrator tissue or cellular biological samples expressing the TF antigen.

Another embodiement of the present invention refers to a method for *in vitro* diagnostics of MSI from a biological sample characterized by comprising, the steps of:
a) Sectioning a biological sample obtained in vitro, into sections of formalin fixed paraffin embedded (FFPE) tissue blocks of 3 um sections, mounting onto glass slides, dewaxing with 100% xylene and rehydrating in 100%; 950; 700; or 50% ethanol washes and submersion in distilled water;
b) Inactivating Endogenous peroxidases with 3% hydrogen peroxide (H2O2) in methanol.
c) Blocking for 30 minutes with normal rabbit serum in PBS with 10% Bovine Serum Albumin (BSA).
d) Contacting the sections with an agent having affinity for the TF antigen selected from the group consisting of: antibodies for TF antigen, fragments of the variable domain of said antibodies or lectins having affinity for the TF antigen and incubating the said sections for 1 hour or overnight at temperatures between 25°C and 4°C;
e) Incubating with a biotin-labeled secondary antibody for 30 minutes;
f) Incubating with an avidin-biotin complex (ABC) reagent for additional 30 minutes;
g) Staining the said sections by 3,3'-diaminobenzidine tetrahydrochloride (DAB) and counterstaining with Gill's hematoxylin solution and applying a coverslip over mounting medium;
h) Examining the sections using an Optical Microscope and estimating the proportion of positive cancer cells within the tumor for classification into positive or negative MSI diagnosis with a cut-off between 5% and 250, most preferably 5% of cancer cell positivity for TF staining.

In another embodiement, the *in vitro* biological sample to be tested trough the above mentioned method comprises frozen tissue or cells in liquid cytology, gastric washes, vesical washings or urine, ascitic pleural or cerebrospinal fluid or in a diluted fecal sample.

In another embodiment, a method for *in vitro* diagnostics of MSI from an *in vitro* biological sample is characterized by comprising the steps of:
a) Obtaining an in vitro serum or plasma biological sample; and b) Performing an Enzyme-linked-Immunosorbent Assay (ELISA) for detecting the TF antigen employing an anti-TF antibody, selected from the group consisting of: 3C9, SPM320, A68-B/A11, A78/G-A7, B79-H/B8, C80-I/C9 and A68-B/A11; fragments of the variable domain of said antibodies or the lectin, Jacalin.

In another embodiement, the said ELISA is characterized by being directed at the detection of levels of autoantibodies against the TF antigen.

The use, compositions, methods and kit may be advantageously applied in cancer patients including, but not limited to gastric and colorectal carcinoma to significantly facilitate the *in vitro* diagnostics of MSI in the clinical setting through a single marker-based simple, rapid, sensitive and specific antibody-assay for diagnostics, prognostics or prediction of response to immunotherapy treatment.

### Description of the Invention

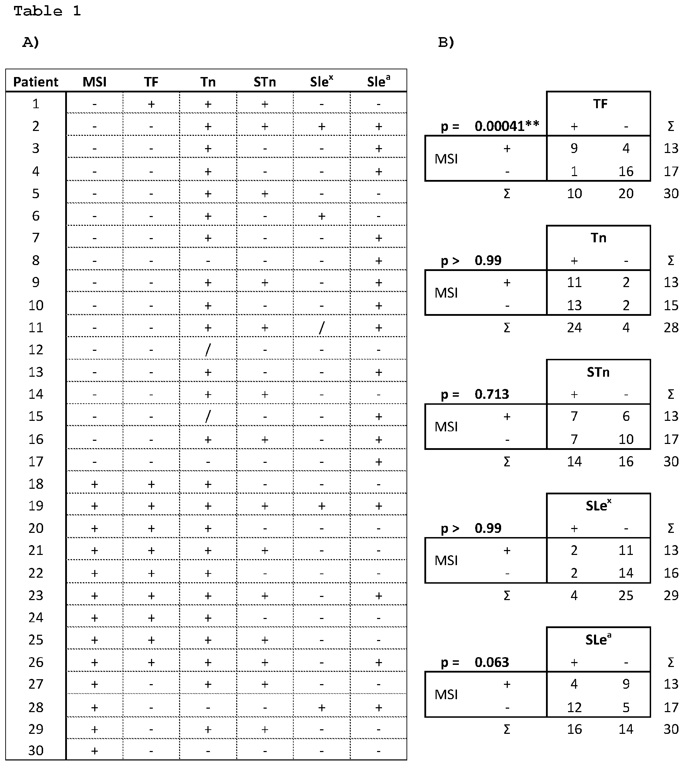

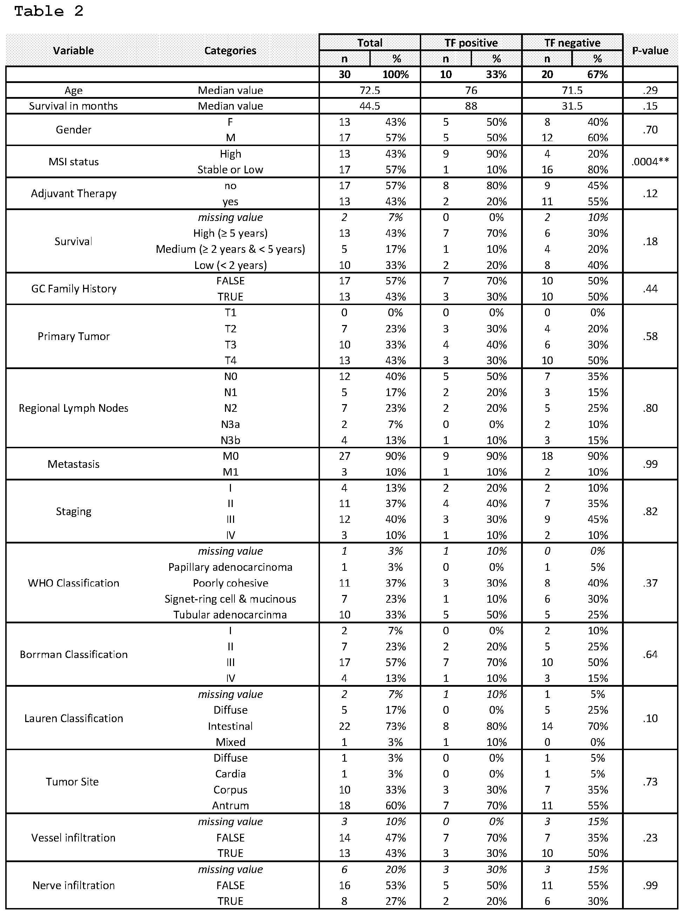

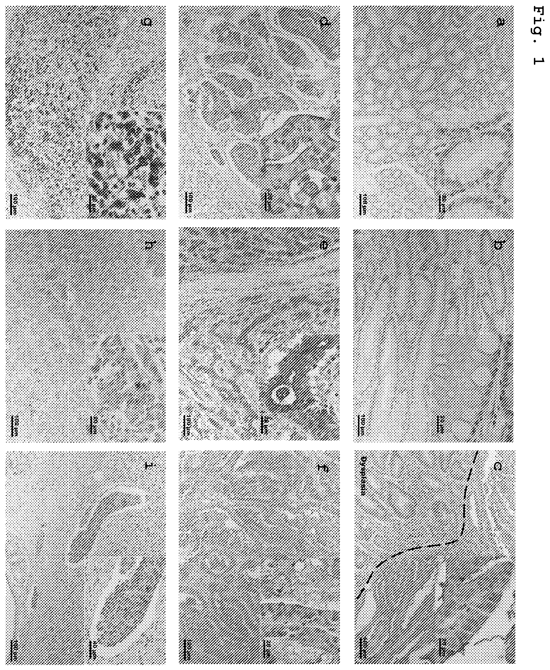

The present invention discloses the analysis of the expression of the TF antigen, amongst five cancer-associated glycan epitopes, in carcinomas with MSI high (positive) and MSI low or stable status (negative), revealing a novel, highly significant association between expression of the TF antigen and MSI status (Tables 1 and 2), without the undesired detection in normal or nonmalignant lesions (Figure 1).

Thus, the present invention discloses the use of antibodies for the TF antigen or other agents with affinity for the TF antigen. Disclosed but not claimed is a composition employed in a method or kit for detecting Microsatellite Instability (MSI) in tumor tissue samples including, but not limited to gastric, cancer.

Possible specific antibodies that can be used for the purpose comprise the TF antigen antibodies 3C9, SPM320, A68/B-A11, A78/G-A7, B79-H/B8, C80-I/C9, A68-B/A11, all characterized by having affinity for the TF antigen, that can be obtained from commercial sources, such as Novus Biologicals, Invitrogen, Cell Sciences, Creative Diagnostics, Creative Biolabs the Abcam, Santa Cruz, Histosearch, Life Span Biosciences, Creative Diagnostics, or other companies such as Abnova, Raybiotech, NSJ Reagents, MyBiosource, American Research Products Inc, Fitzgerald Industries International and Progen.

In other embodiments of the present invention, fragments of the variable domain of the above mentioned antibodies can be used for the purpose of this invention.

In other embodiments of the present invention, lectins with high affinity for the TF antigen, such as Jacalin can be used for the purpose of this invention.

The said antibodies, antibody fragments or lectins are used in a composition for detecting MSI.

The composition for detecting MSI is prepared by diluting a specific antibody, antibody fragment or lectin with affinity for the TF antigen in Phosphate Buffered Saline (PBS) with 20 (w/v) Bovine Serum Albumine (BSA), in amounts ranging from 5-50 micrograms, most preferably 33 micrograms.

Using the said composition, it is possible to develop an embodiment of the present invention that refers to a method for *in vitro* diagnostics of MSI, on the basis of the expression of TF antigen (Figure 1), which comprises the following steps:
a) A biological sample obtained in vitro, such as formalin fixed paraffin embedded (FFPE) tissue blocks is cut into 3um sections, mounted onto glass slides, dewaxed using 100% xylene and rehydrated in 100%; 950; 700; 50% ethanol washes and submersion in distilled water.
b) Endogenous peroxidases are inactivated with 3% hydrogen peroxide (H2O2) in methanol;
c) Tissue sections are blocked for 30 minutes with normal rabbit serum in PBS with 10% Bovine Serum Albumin (BSA);
d) The said compositions comprising antibodies, antibody fragments or lectins specific for the TF antigen are contacted and incubated with the said tissue sections for 1 hour or overnight, at temperatures between 25°C and 4°C, respectivelly, most preferably overnight at 4°C;
e) Biotin-labeled secondary antibody is applied for 30 minutes;
f) The ABC kit from Vector Labs, or other of the kind is applied for additional 30 minutes;
g) Sections are stained by 3,3'-diaminobenzidine tetrahydrochloride (DAB) and counterstained with Gill's hematoxylin solution;
h) Slides are examined under a Microscope and the proportion of positive cancer cells within the tumor are estimated for the classification into MSI positive or MSI negative categories, with a cut-off between 5% and 250, most preferably 50 of cancer cell positivity for TF.

By employing this method in 30 gastric carcinoma cases (13 MSI positive and 17 MSI negative by current PCR-based analysis)a striking and highly significant association between the expression of TF and MSI status is obtained (p < 0.001; Fisher's exact test). Among the 10 TF positive cases, 9 are MSI positive, which suggests an unprecedented specificity of the TF antigen for this gastric cancer molecular subtype (Table1). Results of employing the method indicate that the TF antigen also excels regarding sensitivity, as 16 of the 20 TF negative cases were MSI negative (Table 1). This results in sensitivity and specificity values of 69.2% (9/13) and 94.10 (16/17), respectively. Positive and negative predictor values are 900 (9/10) and 800 (16/20), respectively.

Another advantage of the method is that the mucosa adjacent to tumors, including normal glands and highly inflamed regions, as well as intestinal metaplasia and dysplasia, are completely negative, underlining the absence of this glycan marker in nonmalignant and pre-malignant conditions (Fig.1 a-c). Among the positive cases, the staining is typically membranous and found on average in around 300 of all cancer cells of the tumor. In well-differentiated gastric carcinomas the staining is typically at the apical membrane and included secretion (Fig1 d-f). The subcellular localization among poorly differentiated gastric carcinomas shifted typically to cytoplasmic (Fig1 g-j). The expression of TF seems to associate with good prognosis for the patients, reflected by the increased median patient survival (88 months vs 31.5 months) and the high proportion of patients being alive 5 years after diagnosis (70% vs 30%) (Table2). This improved prognosis is in accordance with expectations, as an on average better survival is known to occur for MSI patients.

In other embodiments of the present invention's method for detecting MSI, the said biological sample may comprise frozen tissue or tumor cells in liquid cytology, gastric washes, vesical washings or urine, ascitic pleural or cerebrospinal fluid or in a diluted fecal sample.

Disclosed but not claimed is a kit comprising the above mentioned compositions for detecting MSI by conducting the present method invention.

It is further disclosed that the said kit further comprises control and/or calibrator tissue or cellular biological samples expressing the TF antigen.

In summary, the use, compositions, methods and kit may be advantageously applied in the in vitro diagnostics of MSI in carcinoma of gastric, colorectal and other tissues to significantly improve the identification of this distinct cancer subtype in the clinical setting through a single marker-based, rapid, sensitive and specific antibody-assay for prognostics or prediction of response to treatment of cancer patients.

### Brief Description of the Figures

**Table 1:** Describes the association analysis of aberrant glycan epitopes with MSI status. A) The expression of five markers of aberrant glycosylation, namely the Thomsen-Friedenreich (TF, Galβ1-3GalNAcα1) antigen, the Thomsen-nouvelle (Tn, GalNAcα1) and its sialylated form - sialyl Tn (STn, Neu5Acα2-3GalNAcα1), sialyl-Lewis A (SLea, Neu5Acα2-3Galβ1-3[Fucα1-4]GlcNAcβ-) and sialyl-Lewis X (SLex, Neu5Acα2-3Galβ1-4[Fucα1-3]GlcNAcβ-)was evaluated in 13 MSI positive and 17 MSI negative gastric carcinomas. B) Statistical analysis of markers with MSI was performed using Fisher's exact test, revealing a highly significant association of TF with MSI(**). Missing values are indicated by (/).
**Table 2:** Describes the clinicopathological analysis of gastric cancer patients according to TF antigen status. A total cohort of 30 patients, comprising TF positive patients (10) and TF negative patients (20), along with comparison of the TF status (negative/positive) with clinicopathological features. Associations between clinicopathologic groups and the two TF marker status groups were calculated using Fisher's exact test for features with two categories and χ² test for features with more than three categories. Difference in medians of age and survival since diagnosis (in months) of the TF status groups was statistically evaluated using Mann-Whitney-Wilcoxon test. P-values ≤ 0.05 were considered to be statistically significant (**).
**Figure 1****:** Representative staining images of the Thomsen-Friedenreich (TF) antigen expression in human gastric tissue samples.(a) Gastric mucosa is negative for TF, here represented by histologically normal mucosa adjacent to MSI positive and TF positive tumor. (b) Representative staining image of intestinal metaplasia showing that even goblet cells, which are commonly enriched in truncated O-glycan carriers, were negative for TF. (c) TF negative dysplasia next to TF positive carcinoma, showing the specificity of TF for malignant cells. (d-f) Well-differentiated gastric carcinomas show a typical membranous TF staining at the apical surface, including mucinous secretions. (g-i) Poorly differentiated gastric carcinomas were dominated by cytoplasmic staining. (i) TF positive carcinoma cells within an infiltrated vessel.

### EXAMPLES

### Example 1 - Preparation of the compositions for detecting MSI comprising antibodies for the TF antigen

In one example of the invention, a composition for *in vitro* diagnostics of MSI is produced by diluting an antibody against the TF antigen in Phosphate Buffered Saline (PBS) with 20 (w/v) of Bovine Serum Albumine (BSA), in amounts ranging from 5-100 micrograms.

### Example 2 - Preparation of the compositions for detecting MSI comprising antibody or antigen receptor fragments

In one embodiment of the invention, a composition for *in vitro* diagnostics of MSI comprises fragments of variable regions targeting the TF antigen, including, but not limited to, the variable chains of the heavy or the light immunoglobulin chains, as well as the variable chains of T-Cell receptors.

### Example 3 - Preparation of the compositions for detecting MSI comprising lectins

In one embodiment of the invention, a composition for *in vitro* diagnostics of MSI comprises lectins with high affinity for the TF antigen, including, but not limited to, Jacalin and mixtures thereof.

### Example 4 - Method for in vitro diagnostics of MSI in FFPE tissue samples

In one example, a method employing the compositions prepared as described in the previous examples comprises the steps of:
a) Sectioning a biological sample obtained in vitro, such as formalin fixed paraffin embedded (FFPE) tissue blocks into 3 um sections, mounting onto glass slides, dewaxing with 100% xylene and rehydrating in 100%; 950; 700; 50% ethanol washes and submersion in distilled water;
b) Inactivating Endogenous peroxidases with 3% hydrogen peroxide (H2O2) in methanol.
c) Blocking for 30 minutes with normal rabbit serum in PBS with 10% Bovine Serum Albumin (BSA).
d) Contacting the compositions described in claims 2-12 and incubating the said sections for 1 hour or overnight at temperatures between 25°C and 4°C, respectivelly, most preferably overnight at 4°C;
e) Incubating with a biotin-labeled secondary antibody for 30 minutes;
f) Incubating with the ABC kit from Vector Labs, or others of the sort, for additional 30 minutes;
g) Staining the said sections by 3,3'-diaminobenzidine tetrahydrochloride (DAB) and counterstaining with Gill's hematoxylin solution and appllying a coverslip over mounting medium;
h) Examining the slides using a Microscope and estimating the proportion of positive cancer cells within the tumor for classification into positive or negative MSI diagnosis with a cut-off of over 50 of cancer cell positivity for TF antigen staining.

The advantage of this method is that through the expression of the TF antigen, as a single marker, a highly reliable predictor of MSI status in cancer is achieved, and thus, the application of TF antigen measurement has therefore huge potential for the stratification of patients with MSI in a time and cost efficient manner.

### Example 5 - Method for in vitro diagnostics of MSI in cellular samples

In another example, the biological sample to be employed in the method described in the previous example can be frozen tissues or comprise cells imprinted and fixed onto glass slides derivedfrom liquid cytology; gastric washes; vesical washings or urine; ascitic pleural or cerebrospinal fluid or from a diluted fecal sample. The advantage of this celular samples is that they may be obtainef from minimally invasive procedures that din't require surgery for oavtainin tissue biopsies or surgical specimens.

### Example 6 - Serological methods for in vitro diagnostics of MSI

Another example regards the applicability of a TF antigen detecting ELISA (Enzyme-linked-Immunosorbent Assay) based serological assay for the diagnosis of the MSI subtype which has potential utility in the clinical setting.

Thus, in another embodiements a method for *in vitro* diagnostics of MSI may be developed comprising the steps of:
a) Obtaining an *in vitro* serum or plasma biological sample;
b) Performing an ELISA for detecting the TF antigen employing one of the compositions described in claims 2-12.

### Example 7 - Serological methods for in vitro diagnostics of MSI

In another embodiement, serological methods described in the previous example can follow a strategy such as the detection of elevated levels of autoantibodies against TF, which can have indicative properties for the MSI status.

### References

1. Cancer Genome Atlas Research, N. (2014) Comprehensive molecular characterization of gastric adenocarcinoma. Nature. 513, 202-209
2. Cristescu, R., Lee, J., Nebozhyn, M., Kim, K. M., Ting, J. C., Wong, S. S., Liu, J., Yue, Y. G., Wang, J., Yu, K., Ye, X. S., Do, I. G., Liu, S., Gong, L., Fu, J., Jin, J. G., Choi, M. G., Sohn, T. S., Lee, J. H., Bae, J. M., Kim, S. T., Park, S. H., Sohn, I., Jung, S. H., Tan, P., Chen, R., Hardwick, J., Kang, W. K., Ayers, M., Hongyue, D., Reinhard, C., Loboda, A., Kim, S. and Aggarwal, A. (2015) Molecular analysis of gastric cancer identifies subtypes associated with distinct clinical outcomes. Nature medicine. 21, 449-456
3. Polom, K., Boger, C., Smyth, E., Marrelli, D., Behrens, H. M., Marano, L., Becker, T., Lordick, F., Rocken, C. and Roviello, F. (2018) Synchronous metastatic gastric cancer-molecular background and clinical implications with special attention to mismatch repair deficiency. European journal of surgical oncology : the journal of the European Society of Surgical Oncology and the British Association of Surgical Oncology. 44, 626-631
4. Polom, K., Marano, L., Marrelli, D., De Luca, R., Roviello, G., Savelli, V., Tan, P. and Roviello, F. (2018) Meta-analysis of microsatellite instability in relation to clinicopathological characteristics and overall survival in gastric cancer. The British journal of surgery. 105, 159-167
5. Pinto, M., Oliveira, C., Machado, J. C., Cirnes, L., Tavares, J., Carneiro, F., Hamelin, R., Hofstra, R., Seruca, R. and Sobrinho-Simoes, M. (2000) MSI-L gastric carcinomas share the hMLH1 methylation status of MSI-H carcinomas but not their clinicopathological profile. Lab Invest. 80, 1915-1923
6. Polom, K., Marrelli, D., Roviello, G., Voglino, C., Vindigni, C., Generali, D. and Roviello, F. (2017) Single Center Experience on Anatomy-and Histopathology-Based Gastric Cancer Molecular Classification. Cancer investigation. 35, 325-332
7. Le, D. T., Uram, J. N., Wang, H., Bartlett, B. R., Kemberling, H., Eyring, A. D., Skora, A. D., Luber, B. S., Azad, N. S., Laheru, D., Biedrzycki, B., Donehower, R. C., Zaheer, A., Fisher, G. A., Crocenzi, T. S., Lee, J. J., Duffy, S. M., Goldberg, R. M., de la Chapelle, A., Koshiji, M., Bhaijee, F., Huebner, T., Hruban, R. H., Wood, L. D., Cuka, N., Pardoll, D. M., Papadopoulos, N., Kinzler, K. W., Zhou, S., Cornish, T. C., Taube, J. M., Anders, R. A., Eshleman, J. R., Vogelstein, B. and Diaz, L. A., Jr. (2015) PD-1 Blockade in Tumors with Mismatch-Repair Deficiency. The New England journal of medicine. 372, 2509-2520
8. Smyth, E. C., Wotherspoon, A., Peckitt, C., Gonzalez, D., Hulkki-Wilson, S., Eltahir, Z., Fassan, M., Rugge, M., Valeri, N., Okines, A., Hewish, M., Allum, W., Stenning, S., Nankivell, M., Langley, R. and Cunningham, D. (2017) Mismatch Repair Deficiency, Microsatellite Instability, and Survival: An Exploratory Analysis of the Medical Research Council Adjuvant Gastric Infusional Chemotherapy (MAGIC) Trial. JAMA oncology. 3, 1197-1203
9. Pinho, S. S. and Reis, C. A. (2015) Glycosylation in cancer: mechanisms and clinical implications. Nature reviews. Cancer. 15, 540-555
10. Clausen, H., Stroud, M., Parker, J., Springer, G. and Hakomori, S. (1988) Monoclonal antibodies directed to the blood group A associated structure, galactosyl-A: specificity and relation to the Thomsen-Friedenreich antigen. Molecular immunology. 25, 199-204
11. Cao, Y., Stosiek, P., Springer, G. F. and Karsten, U. (1996) Thomsen-Friedenreich-related carbohydrate antigens in normal adult human tissues: a systematic and comparative study. Histochemistry and cell biology. 106, 197-207
12. David, L., Nesland, J. M., Clausen, H., Carneiro, F. and Sobrinho-Simoes, M. (1992) Simple mucin-type carbohydrate antigens (Tn, sialosyl-Tn and T) in gastric mucosa, carcinomas and metastases. APMIS. Supplementum. 27, 162-172
13. Reis, C. A., Osorio, H., Silva, L., Gomes, C. and David, L. (2010) Alterations in glycosylation as biomarkers for cancer detection. Journal of clinical pathology. 63, 322-329
14. Springer, G. F. (1984) T and Tn, general carcinoma autoantigens. Science. 224, 1198-1206
15. Pedersen, J. W. and Wandall, H. H. (2011) Autoantibodies as Biomarkers in Cancer. Laboratory Medicine. 42, 623-628
16. Le, D. T., Durham, J. N., Smith, K. N., Wang, H., Bartlett, B. R., Aulakh, L. K., Lu, S., Kemberling, H., Wilt, C., Luber, B. S., Wong, F., Azad, N. S., Rucki, A. A., Laheru, D., Donehower, R., Zaheer, A., Fisher, G. A., Crocenzi, T. S., Lee, J. J., Greten, T. F., Duffy, A. G., Ciombor, K. K., Eyring, A. D., Lam, B. H., Joe, A., Kang, S. P., Holdhoff, M., Danilova, L., Cope, L., Meyer, C., Zhou, S., Goldberg, R. M., Armstrong, D. K., Bever, K. M., Fader, A. N., Taube, J., Housseau, F., Spetzler, D., Xiao, N., Pardoll, D. M., Papadopoulos, N., Kinzler, K. W., Eshleman, J. R., Vogelstein, B., Anders, R. A. and Diaz, L. A., Jr. (2017) Mismatch repair deficiency predicts response of solid tumors to PD-1 blockade. Science. 357, 409-413.
Lisbon, May 3^{rd}, 2019

## Claims

1. Use of an agent having affinity for the Thomsen-Friedenreich (TF) antigen selected from the group consisting of: antibodies for TF antigen, fragments of the variable domain of said antibodies or lectins having affinity for the TF antigen as reagents for *in vitro* diagnostics of Microsatelite Instability (MSI).

2. Use according to claim 1, wherein the agent having affinity for the Thomsen-Friedenreich (TF) antigen is the anti-TF antigen antibody 3C9.

3. Use according to claim 1, wherein the agent having affinity for the Thomsen-Friedenreich (TF) antigen is the anti-TF antigen antibody SPM320.

4. Use according to claim 1, wherein the agent having affinity for the Thomsen-Friedenreich (TF) antigen is the anti-TF antigen antibody A68-B/A11.

5. Use according to claim 1, wherein the agent having affinity for the Thomsen-Friedenreich (TF) antigen is the anti-TF antigen antibody A78/G-A7.

6. Use according to claim 1, wherein the agent having affinity for the Thomsen-Friedenreich (TF) antigen is the anti-TF antigen antibody B79-H/B8.

7. Use according to claim 1, wherein the agent having affinity for the Thomsen-Friedenreich (TF) antigen is the anti-TF antigen antibody C80-I/C9.

8. Use according to claim 1, wherein the agent having affinity for the Thomsen-Friedenreich (TF) antigen is the anti-TF antigen antibody A68-B/A11.

9. Use according to claim 1, wherein the agent having affinity for the Thomsen-Friedenreich (TF) antigen is a fragment of the variable domain of an anti-TF antibody, selected from the group consisting of: 3C9, SPM320, A68-B/A11, A78/G-A7, B79-H/B8, C80-I/C9 and A68-B/A11.

10. Use according to claim 1, wherein the agent having affinity for the Thomsen-Friedenreich (TF) antigen is the lectin, Jacalin.

11. A Method for in vitro diagnostics of MSI from an in vitro biological sample **characterized by** comprising, the steps of:
a) Sectioning a biological sample obtained *in vitro*, into sections of formalin fixed paraffin embedded (FFPE) tissue blocks of 3 µm sections, mounting onto glass slides, dewaxing with 100% xylene and rehydrating in 100%; 95%; 70%; or 50% ethanol washes and submersion in distilled water;
b) Inactivating Endogenous peroxidases with 3% hydrogen peroxide (H₂0₂) in methanol;
c) Blocking for 30 minutes with normal rabbit serum in PBS with 10% Bovine Serum Albumin (BSA);
d) Contacting the sections with an agent having affinity for the TF antigen selected from the group consisting of: antibodies for TF antigen, fragments of the variable domain of said antibodies or lectins having affinity for the TF antigen and incubating the said sections for 1 hour or overnight at temperatures between 25°C and 4°C;
e) Incubating with a biotin-labeled secondary antibody for 30 minutes;
f) Incubating with an avidin-biotin complex (ABC) reagent for additional 30 minutes;
g) Staining the said sections by 3,3'-diaminobenzidine tetrahydrochloride (DAB) and counterstaining with Gill's hematoxylin solution and applying a coverslip over mounting medium;
h) Examining the sections using an Optical Microscope and estimating the proportion of positive cancer cells within the tumor for classification into positive or negative MSI diagnosis with a cut-off between 5% and 25%, most preferably 5% of cancer cell positivity for TF staining.

12. Method according to claim 11, wherein in step d) the sections are contacted with an anti-TF antibody, selected from the group consisting of: 3C9, SPM320, A68-B/A11, A78/G-A7, B79-H/B8, C80-I/C9 and A68-B/A11; fragments of the variable domain of said antibodies or the lectin, Jacalin.

13. Method according to claims 11 or 12, where the biological sample comprises frozen tissue or cells in liquid cytology, gastric washes, vesical washings or urine, ascitic pleural or cerebrospinal fluid or in a diluted fecal sample .

14. A Method for in vitro diagnostics of MSI in cancer patients from an in vitro biological sample **characterized by** comprising the steps of:
a) Obtaining an *in vitro* serum or plasma biological sample; and
b) Performing an Enzyme-linked-Immunosorbent Assay (ELISA) for detecting the TF antigen employing an anti-TF antibody, selected from the group consisting of: 3C9, SPM320, A68-B/A11, A78/G-A7, B79-H/B8, C80-I/C9 and A68-B/A11; fragments of the variable domain of said antibodies or the lectin, Jacalin.

15. A method for in vitro diagnostics of MSI according to claim 14 where the said ELISA is **characterized by** comprising the detection of levels of autoantibodies against TF.

## Patentansprüche

1. Anwendung eines Wirkstoffes mit Affinität zum Thomsen-Friedenreich (TF)-Antigen gewählt aus der Gruppe bestehend aus: Antikörper zum TF-Antigen, Fragmente der wechselhaften Domäne von genannten Antikörpern oder Lektinen mit Affinität zum TF-Antigen als Reagens für *in* vitro-Diagnose der Mikrosatelliteninstabilität (MSI).

2. Anwendung gemäß Anspruch 1, wobei der Wirkstoff mit Affinität zum Thomsen-Friedenreich (TF)-Antigen der anti-TF-Antigen-Antikörper 3C9 ist.

3. Anwendung gemäß Anspruch 1, wobei der Wirkstoff mit Affinität zum Thomsen-Friedenreich (TF)-Antigen der anti-TF-Antigen-Antikörper SPM320 ist.

4. Anwendung gemäß Anspruch 1, wobei der Wirkstoff mit Affinität zum Thomsen-Friedenreich (TF)-Antigen der anti-TF-Antigen-Antikörper A68-B/A11 ist.

5. Anwendung gemäß Anspruch 1, wobei der Wirkstoff mit Affinität zum Thomsen-Friedenreich (TF)-Antigen der anti-TF-Antigen-Antikörper A78/G-A7 ist.

6. Anwendung gemäß Anspruch 1, wobei der Wirkstoff mit Affinität zum Thomsen-Friedenreich (TF)-Antigen der anti-TF-Antigen-Antikörper B79-H/B8 ist.

7. Anwendung gemäß Anspruch 1, wobei der Wirkstoff mit Affinität zum Thomsen-Friedenreich (TF)-Antigen der anti-TF-Antigen-Antikörper C80-I/C9 ist.

8. Anwendung gemäß Anspruch 1, wobei der Wirkstoff mit Affinität zum Thomsen-Friedenreich (TF)-Antigen der anti-TF-Antigen-Antikörper A68-B/A11 ist.

9. Anwendung gemäß Anspruch 1, wobei der Wirkstoff mit Affinität zum Thomsen-Friedenreich (TF)-Antigen ein Fragment de werchselhaften Domäne von einem anti-TF-Antikörper ist, gewählt aus der Gruppe bestehend aus: 3C9, SPM320, A68-B/A11, A78/G-A7, B79-H/B8, C80-I/C9 und A68-B/A11.

10. Anwendung gemäß Anspruch 1, wobei der Wirkstoff mit Affinität zum Thomsen-Friedenreich (TF)-Antigen das Jacalin-Lektin ist.

11. Eine Methode für *in* vitro-Diagnose der MSI aus einer biologischen *in* vitro-Probe **gekennzeichnet durch** folgende umfassende Schritte:
a) Zerlegung einer biologischen Probe gewonnen *in vitro* in Abschnitte von Formalin-fixierten Paraffineingebetteten (FFPE) Gewebeblöcken von 3 µm- Abschnitten, Einbau auf Glasobjektträgern, Entwachsung mit 100% Xylene
und Rehydrierung durch Waschen mit 100%-; 95%-; 70%-; oder 50%-igem Ethanol und Eintauchen in destilliertes Wasser;
b) Inaktivierung endogener Peroxidasen mit 3%igem Wasserstoffperoxid (H₂O₂) in Methanol;
c) Blockierung für 30 Minuten mit normalem Kaninchenserum in PBS mit 10%igem Rinderserumalbumin (BSA);
d) Kontaktnahme der Abschnitte mit einem Wirkstoff mit Affinität zum TF-Antigen gewählt aus der Gruppe bestehend aus: Antikörper zum TF-Antigen, Fragmente der wechselhaften Domäne von genannten Antikörpern oder Lektinen mit Affinität zum TF-Antigen und Inkubation der Abschnitte für 1 Stunde oder über Nacht bei Temperaturen zwischen 25°C und 4°C;
e) Inkubation mit einem Biotin-markierten sekundären Antikörper für 30 Minuten;
f) Inkubation mit einem Avidin-Biotin-Komplex-Reagens (ABC) für weitere 30 Minuten;
g) Färbung der besagten Abschnitte mit 3,3'-Diaminobenzidin-Tetrahydrochlorid (DAB) und Gegenfärbung mit Gill'scher Hämatoxylinlösung und Verwendung eines Deckglases über den Einbaumitteln;
h) Untersuchung der Abschnitte mittels Anwendung eines Lichtmikroskopes und Schätzung des Anteils positiver Krebszellen innerhalb des Tumors zur Einstufung in positive oder negative MSI-Diagnose mit einem Grenzwert zwischen 5% und 25%, vorzugsweise 5% positiver Krebszellen für die TF-Färbung.

12. Methode gemäß Anspruch 11, wobei die Abschnitte in Schritt d) mit einem anti-TF-Antikörper gewählt aus der Gruppe bestehend aus: 3C9, SPM320, A68-B/A11, A78/G-A7, B79-H/B8, C80-I/C9 und A68-B/A11; Fragmente der wechselhaften Domäne von genannten Antikörpern oder Jacalin-Lektinen.

13. Methode gemäß Ansprüche 11 oder 12, wobei die biologische Probe gefrorene Gewebe oder Zellen in Flüssigzytologie, Magenspülungen, Vesikelspülungen oder Urin, aszitischer Pleura- oder Cerebrospinalflüssigkeit oder in einer verdünnten Stuhlprobe umfasst.

14. Eine Methode für *in* vitro-MSI-Diagnosen in Krebspatienten aus einer *in vitro* biologischen Probe **dadurch gekennzeichnet**, folgende Schritte umzufassen:
a) Erwerb eines *in* vitro-Serums oder einer biologischen Plasmaprobe; und
b) Durchführung eines Enzyme-linked Immunosorbent Assays (ELISA) zum Nachweis des TF-Antigens unter Verwendung eines anti-TF-Antikörpers Gewählt aus der Gruppe bestehend aus: 3C9, SPM320, A68-B/A11, A78/G-A7, B79-H/B8, C80-I/C9 und A68-B/A11; Fragmente der wechselhaften Domäne von genannten Antikörpern oder Jacalin-Lektinen.

15. Eine Methode für *in* vitro-MSI-Diagnosen gemäß Anspruch 14, wobei genanntes ELISA **dadurch gekennzeichnet ist, dass** Autoantikörpern gegen TF nachgewiesen werden.

## Revendications

1. Utilisation d'un agent ayant une affinité pour l'antigène Thomsen-Friedenreich (TF), sélectionné du groupe qui consiste en ce qui suit : anticorps pour antigène TF, fragments du domaine variable des dits anticorps ou lectines ayant une affinité pour l'antigène TF comme réactifs pour des diagnostiques *in vitro* de l'instabilité microsatellite (MSI).

2. Utilisation selon la revendication 1, où l'agent ayant une affinité pour l'antigène Thomsen-Friedenreich (TF) est l'anticorps 3C9 de l'anti-antigène TF.

3. Utilisation selon la revendication 1, où l'agent ayant une affinité pour l'antigène Thomsen-Friedenreich (TF) est l'anticorps SPM320 de l'anti-antigène TF.

4. Utilisation selon la revendication 1, où l'agent ayant une affinité pour l'antigène Thomsen-Friedenreich (TF) est l'anticorps A68-B/A11 de l'anti-antigène TF.

5. Utilisation selon la revendication 1, où l'agent ayant une affinité pour l'antigène Thomsen-Friedenreich (TF) est l'anticorps A78/G-A7 de l'anti-antigène TF.

6. Utilisation selon la revendication 1, où l'agent ayant une affinité pour l'antigène Thomsen-Friedenreich (TF) est l'anticorps B79-H/B8 de l'anti-antigène TF.

7. Utilisation selon la revendication 1, où l'agent ayant une affinité pour l'antigène Thomsen-Friedenreich (TF) est l'anticorps C80-I/C9 de l'anti-antigène TF.

8. Utilisation selon la revendication 1, où l'agent ayant une affinité pour l'antigène Thomsen-Friedenreich (TF) est l'anticorps A68-B/A11 de l'anti-antigène TF.

9. Utilisation selon la revendication 1, où l'agent ayant une affinité pour l'antigène Thomsen-Friedenreich (TF) est un fragment du domaine variable d'un anti-anticorps TF, sélectionné du groupe qui consiste en ce qui suit : 3C9, SPM320, A68-B/A11, A78/G-A7, B79-H/B8, C80-I/C9 et A68-B/All.

10. Utilisation selon la revendication 1, où l'agent ayant une affinité pour l'antigène Thomsen-Friedenreich (TF) est la lectine Jacaline.

11. Méthode pour des diagnostiques in vitro de la MSI à partir d'un échantillon biologique in vitro **caractérisé en ce qu'**il comprend les étapes suivantes :
a) Sectionnement d'un échantillon biologique obtenu *in vitro*, en sections de blocs de tissus fixés au formol et inclus dans la paraffine (FFIP) de sections de 3 µm, montées sur des lames de verre, avec un déparaffinage à l'aide de 100 % de xylène et une réhydratation à 100 %, 95 %, 70 %, ou 50 % de lavages à l'éthanol et la submersion dans de l'eau distillée ;
b) Désactivation de peroxydases endogènes avec 3 % de peroxyde d'hydrogène (H₂0₂) dans du méthanol ;
c) Blocage pendant 30 minutes à l'aide de sérum de lapin normal en PBS avec 10 % d'albumine de sérum bovin (BSA) ;
d) Contact des sections avec un agent ayant une affinité pour l'antigène TF sélectionné du groupe qui consiste en ce qui suit : anticorps pour antigène TF, fragments du domaine variable des dits anticorps ou lectines ayant une affinité pour l'antigène TF et incubant lesdites sections pendant 1 heure ou pendant la nuit à des températures comprises entre 25° C et 4° C ;
e) Incubation avec un anticorps secondaire labellisé biotine pendant 30 minutes ;
f) Incubation avec un réactif de complexe avidine-biotine (ABC) pendant 30 minutes supplémentaires ;
g) Coloration des dites sections par 3,3'-Diaminobenzidine Tetrahydrochloride (DAB) et contre-colorer à l'aide de solution d'hématoxyline de Gill et appliquant une lamelle sur un montage moyen ;
h) Examen des sections en utilisant un Microscope Optique et en estimant la proportion des cellules cancéreuses positives dans la tumeur pour la classification en diagnostic MSI positif ou négatif avec une coupure entre 5 % et 25 %, surtout de préférence 5 % de la positivité de la cellule cancéreuse pour la coloration TF.

12. Méthode selon la revendication 11, où à l'étape d), les sections sont en contact avec un anti-anticorps TF, sélectionné du groupe qui consiste en ce qui suit : 3C9, SPM320, A68-B/A11, A78/G-A7, B79- H/B8, C80-I/C9 et A68-B/All ; fragments du domaine variable des dits anticorps ou de la lectine Jacaline.

13. Méthode selon les revendications 11 ou 12, où l'échantillon biologique comprend des cellules ou un tissu congelé(s) dans de la cytologie liquide, des lavages gastriques, de l'urine ou des lavages vésicaux, du fluide cérébrospinal ou pleural d'ascite, ou dans un échantillon fécal dilué.

14. Méthode pour des diagnostics in vitro de MSI, chez des patients cancéreux depuis un échantillon biologique in vitro **caractérisé en ce qu'**il comprend les étapes suivantes :
a) Obtention d'un sérum *in vitro* ou d'un échantillon biologique de plasma ; et
b) Réalisation d'un essai d'immuno-absorption enzymatique (ELISA) pour détecter l'antigène TF en employant un anti-anticorps TF, sélectionné du groupe qui consiste en ce qui suit : 3C9, SPM320, A68-B/A11, A78/G-A7, B79-H/B8, C80-I/C9 et A68-B/A11 ; fragments du domaine variable des dits anticorps ou la lectine Jacaline.

15. Méthode pour des diagnostics in vitro de MSI selon la revendication 14 où ledit ELISA se **caractérise en ce qu'**il comprend la détection de niveaux d'auto-anticorps contre TF.
